# EUROPEAN PATENT APPLICATION

(11) **EP 3 485 895 A1**
(43) Date of publication of application: **22.05.2019**
(21) Application number: 17813449.0
(22) Date of filing: 19.06.2017
(51) Int. Cl.: A61K 38/02, A61K 48/00, A61P 1/04, A61P 1/16, A61P 9/00, A61P 9/12, A61P 11/00, A61P 13/12, A61P 19/02, A61P 21/02, A61P 25/02, A61P 25/16, A61P 25/28, A61P 29/00, A61P 35/00, A61P 35/02, A61P 43/00, C07K 14/47

(54) **TGF INHIBITOR CONTAINING REIC/Dkk-3 PROTEIN AS ACTIVE INGREDIENT**

(30) Priority: 17.06.2016 JP 2016121187
(71) Applicant: Momotaro-Gene Inc., Okayama 700-0904 (JP)
(72) Inventor: KINOSHITA Rie, Okayama-shi Okayama 700-8530 (JP); FUTAMI Junichiro, Okayama-shi Okayama 700-8530 (JP); KUMON Hiromi, Okayama-shi Okayama 700-0904 (JP)
(74) Representative: Grünecker Patent- und Rechtsanwälte PartG mbB
(86) International application number: PCT/JP2017/022588
(87) International publication number: WO 2017/217556

(57) **Abstract**

For purposes of providing a pharmaceutical to be used for preventing or treating a TGFβ-related disease by inhibiting signaling of TGFβ, the present invention provides a TGFβ inhibitor comprising, as an active ingredient, REIC/Dkk-3 protein or a partial protein thereof comprising a Cys-rich domain thereof, or a DNA encoding any of these proteins, and an agent for preventing or treating a TGFβ-related disease containing the TGFβ inhibitor.

## Description

### Technical Field

The present invention relates to a Transforming Growth Factor β (TGFβ) inhibitor for reducing activity of TGFβ in a tissue or an organ, and can improve pathological conditions of TGFβ-related diseases by inhibiting TGFβ.

### Background Art

REIC, which is a secretory protein universally expressed in a large number of normal tissues of a human, is not expressed in a large number of cancer cells, and is named for Reduced Expression in Immortalized Cell (REIC) in 2000 for the background of the discovery. Besides, owing to structural characteristics, it is a member of the Dickkopf (Dkk) family and is generically called REIC/Dkk-3. When a cancer cell is infected with an adenoviral vector (Ad-REIC) forcedly expressing this REIC/Dkk-3 protein, apoptosis derived from endoplasmic reticulum stress is induced in the cancer cell. Since the apoptosis induction is a phenomenon specific to a cancer cell but not found in a normal cell, clinical research on gene therapy for cancer has been started, and its effectiveness and safety have been currently confirmed. On the other hand, there were a large number of unsolved questions regarding functions of the REIC protein extracellularly secreted. It was discovered, in 2008, that the REIC protein induces dendritic cell-like differentiation from a monocyte in blood, and it was found that this activity increases anticancer immune activity (see Patent Literature 1). Besides, it was discovered, in 2010, that the induction of the anticancer immune activity is concentrated in a Cys-rich domain having a molecular weight of 17 kDa included in the REIC protein (see Patent Literature 2), and thus, a minimal domain of medicinal properties of the REIC protein was found.

In recent years, study of tumor immunology has been developed. An "immune checkpoint inhibitor" found in 2010 or later, such as an anti-PD-1 antibody, an anti-CTLA-4 antibody or an anti-CCR4 antibody, releases immunosuppression in a tumor local site, and life prolongation far superior to that attained by chemotherapy has been achieved in clinical trial performed for melanoma and lung cancer.

A treatment for releasing the immunosuppression in a tumor local site to reactivate tumor immune response will definitely become a principal pillar in the future as an alternative in cancer treatment with enhanced QOL. According to some prediction, it is estimated that an immune checkpoint inhibitor will be administered to 60% of advanced cancer patients in the next 10 years (see Non Patent Literature 1). A response rate of the preceding immune checkpoint inhibitor (the anti-PD-1 antibody) is, however, said to be 20 to 30%. The response rate is expected to be further improved in a tumor local site in which a plurality of immunosuppressive molecules are involved when a biopharmaceutical simultaneously blocking a plurality of interaction points is used together.

The secretory protein of the TGFβ exhibits a variety of bioactivities, and it is known that a regulatory T cell (Treg cell) is induced by the TGFβ secreted from a cancer cell or the like in a tumor local site so as to induce immunosuppression.

### Citation List

### Patent Literature

Patent Literature 1: WO2009/119874
Patent Literature 2: WO2012/002582

### Non Patent Literature

Non Patent Literature 1: Ledford H. Nature 508, 7494, 24-26 (2014)

### Summary of Invention

### Technical Problem

An object of the present invention is to provide a pharmaceutical that prevents or treats a TGFβ-related disease by inhibiting signaling of TGFβ. More particularly, an object is to provide a pharmaceutical capable of restoring/activating anticancer immune activity by binding to a TGFβ receptor to inhibit Treg induction activity of TGFβ.

### Solution to Problem

The present inventors have searched for a substance with which REIC/Dkk-3 protein extracellularly interacts. As a result, a TGFβ receptor has been specified as a molecule with which the REIC/Dkk-3 protein interacts. It has been a long-standing problem to identify an interacting molecule of extracellular secreted REIC/Dkk-3 protein and to clarify its action mechanism. The present inventors have mass-produced recombinant protein to construct an evaluation system in which even a low-affinity interaction could be detected, resulting in succeeding in identification and verification of a target molecule. The present inventors have found that REIC/Dkk-3 protein inhibits TGFβ from binding to a TGFβ receptor and inhibits signaling of the TGFβ from the function of TGFβ. This finding reveals that the REIC/Dkk-3 can be used as a cancer immunotherapeutic for restoring/activating tumor immune response by releasing immunosuppression in a tumor local site. In other words, the REIC/Dkk-3 protein can restore/activate the anticancer immune activity by binding to a TGFβ receptor to inhibit regulatory T cell (Treg) inducing activity of the TGFβ. Such activation of the anticancer immune activity exhibited by the REIC protein is similar to a function of an immune checkpoint inhibitor, and it is revealed that the REIC/Dkk-3 is an extremely promising molecule also as a protein preparation usable for prevention or treatment of cancer.

Besides, the present inventors have found that the REIC/Dkk-3 can be used for prevention or treatment of a TGFβ-related disease through the inhibition of signaling of the TGFβ.

Examples of use of the REIC protein based on the present invention include the following:
(1) An immunomodulator for releasing immunosuppression of a tumor local site. When it is used in combination with an existing immune checkpoint inhibitor, cancer immunotherapy is improved.
(2) The action mechanism having succeeded in Ad-REIC treatment, which is REIC/Dkk-3 gene therapy using an adenoviral vector, is identified as a synergetic effect of a "priming effect" of exposing a cancer antigen through apoptosis induction by Ad-REIC and a "booster effect" of activating a dendritic cell or the like in a tumor local site by secreted REIC protein to activate tumor immune response. Regarding exhibition of the latter booster effect, since a mechanism involving regulatory T cell (Treg) induction inhibition through inhibition of signaling of the TGFβ has been clarified, it has become possible to replace the priming function of exposing a cancer antigen with an existing anticancer agent, radiation therapy or the like and to replace the latter booster effect with a REIC protein preparation, resulting in enlargement of the range of choices in cancer treatment.
(3) Various functions of the TGFβ are correlated with various diseases including not only cancer but also, for example, fibrosis of an organ or a tissue. There is a possibility that the effect of the REIC of gently inhibiting the TGFβ activity may improve various pathological conditions, and hence it is a significant drug target.

Specifically, the present invention provides the following:
[1] A TGFβ inhibitor, comprising, as an active ingredient, REIC/Dkk-3 protein or a partial protein thereof comprising a Cys-rich domain thereof, or a DNA encoding any of the proteins.
[2] The TGFβ inhibitor according to [1], comprising the REIC/Dkk-3 protein or the partial protein thereof comprising the Cys-rich domain thereof, the TGFβ inhibitor inhibiting a reaction between TGFβ and a receptor of the TGFβ concentration-dependently in a concentration region corresponding to an active dose of 50 nM or more.
[3] The TGFβ inhibitor according to [1] or [2], the TGFβ inhibitor not completely inhibiting a reaction between TGFβ and a receptor of the TGFβ even when an active dose is high.
[4] The TGFβ inhibitor according to [3], wherein the TGFβ inhibitor does not completely inhibit the reaction between the TGFβ and the receptor even when the active dose is 375 nM or more.
[5] An anti-tumor immune activating agent, comprising the TGFβ inhibitor according to any one of [1] to [4].
[6] An agent for preventing or treating a TGFβ-related disease, comprising the TGFβ inhibitor according to any one of [1] to [4].
[7] The agent for preventing or treating a TGFβ-related disease according to [6], the agent affecting an immune system cell to inhibit a reaction between TGFβ and a receptor of the TGFβ in the immune system cell.
[8] The agent for preventing or treating a TGFβ-related disease according to [6] or [7], reduce adverse reaction otherwise caused by inhibition of TGFβ by not completely inhibiting a reaction between TGFβ and a receptor of the TGFβ.
[9] The agent for preventing or treating a TGFβ-related disease according to any one of [6] to [8], the agent inhibiting a reaction between TGFβ and a receptor of the TGFβ concentration-dependently at a concentration corresponding to an active dose of 50 nM or more, and not completely inhibiting the reaction between the TGFβ and the receptor even at a high concentration of 375 nM or more.
[10] The agent for preventing or treating a TGFβ-related disease according to any one of [6] to [9], wherein the TGFβ-related disease is cancer, and the agent activates anti-tumor immunity.
[11] The agent for preventing or treating a TGFβ-related disease according to [10], wherein the agent releases immunosuppression which mediated by a regulatory T cell induced by TGFβ secreted from a cancer cell, and the agent restores and activates attack of an immune cell against a cancer cell.

This application claims the benefit of priority to Japanese Patent Application No. 2016-121187, the entire contents of which are incorporated herein by reference.

### Advantageous Effects of Invention

It has been concluded that REIC/Dkk-3 protein binds to an extracellular domain of TGFβ receptors I and II with low affinity and that a mechanism to competitively inhibit the bioactivities of TGFβ can work. The rationality of drug development of REIC already clinically developed as gene therapy (Ad-REIC) has been verified. Also the whole picture of its anticancer immune function has been clarified, and it can be applied as a protein preparation. When REIC/Dkk-3 protein is used in combination with an existing immune checkpoint inhibitor or in combination with an existing anticancer agent, radiation therapy or the like, tumor immune response can be restored/activated, and hence more effective cancer immunotherapy can be realized.

### Brief Description of Drawings

Figure 1 is a schematic diagram illustrating a structure of REIC/Dkk-3 protein.
Figure 2 is a diagram of a nucleotide sequence and an amino acid sequence of TGFβRI-ECD. In this drawing, an italic portion corresponds to a secretion signal sequence, and an underlined portion corresponds to a sequence of TGFβRI-ECD. His Tag is attached on the C-terminal side.
Figures 3A and 3B are diagrams illustrating results of a binding experiment in a co-expression system of TGFβRI and FL-REIC, REIC-C domain or Cys-rich domain protein (REIC-17KDa). Figure 3A illustrates a binding method, and Figure 3B illustrates a result of SDS-PAGE.
Figure 4 is a diagram illustrating results of a binding experiment in a co-expression system of REIC-Dkk-3 protein and TGFβRI-Fc or TGFβRII-Fc.
Figure 5 is a diagram illustrating results of a binding experiment of REIC protein to TGFβRI-Fc or TGFβRII-Fc performed by ELISA.
Figure 6 is a diagram illustrating results of a competitive inhibition experiment of TGFβ signal to an EL4 cell by REIC/Dkk-3 protein.
Figure 7 is a diagram illustrating results of a bioassay performed for verifying competitive inhibition of immunoregulatory factor TGFβ by REIC/Dkk-3 protein.
Figure 8 is a diagram illustrating a DNA sequence and an amino acid sequence thereof to be used in using a Cys-rich domain protein as a recombinant protein.
Figure 9 is a diagram illustrating results of a competitive inhibition experiment of TGFβ signal in an EL-4 cell and human fibroblast by REIC/Dkk-3 protein.
Figure 10 is a diagram illustrating a TGFβ pathway inhibitory effect (an effect derived from a reporter gene) by REIC protein.
Figure 11 is a diagram illustrating the TGFβ pathway inhibitory effect (the effect derived from a reporter gene) by REIC protein at each added concentration.

### Description of Embodiment

The present invention will now be described in detail.

The present invention is a TGFβ (transforming growth factor) inhibitor comprising REIC/Dkk-3 protein or a partial protein thereof, or a DNA encoding any of these proteins as an active ingredient. The TGFβ inhibitor is sometimes referred to as the TGFβ signaling inhibitor.

A full-length nucleotide sequence of REIC/Dkk-3 DNA (REIC gene) and an amino acid sequence of a protein encoded by the gene are respectively set forth in SEQ ID NO: 1 and SEQ ID NO: 2. In the amino acid sequence set forth in SEQ ID NO: 2, a sequence consisting of the 1st to the 21st amino acids is presumed to be a signal sequence.

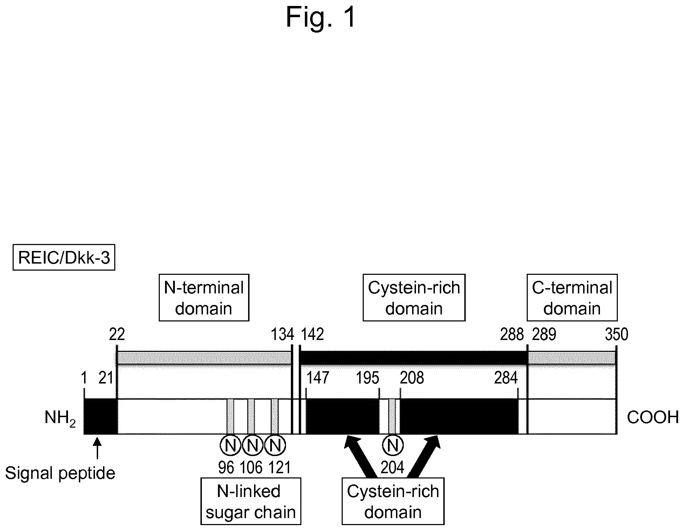

The REIC/Dkk-3 protein has, as illustrated in Figure 1, an N-terminal domain, a Cysteine-rich domain and a C-terminal domain.

The REIC-Dkk-3 protein (REIC protein) of the present invention may be a full-length protein, or may be a partial protein comprising a partial region of the REIC/Dkk-3 protein and comprising at least the Cys-rich domain of the REIC/Dkk-3 protein. The Cys-rich domain is a domain having a molecular weight of 17 kD, and is a minimal domain responsible for immune activity of the REIC/Dkk-3 protein. Examples of such a partial protein include a Cys-rich domain protein (REIC-17kDa (SGE-REIC-17kDa)) and a partial protein ((REIC C domain (SGE-REIC-C)) obtained by deleting 120 amino acids at the N-terminal from the full-length REIC/Dkk-3 protein. The Cys-rich domain protein corresponds to a region consisting of an amino acid sequence from the 135th to the 288th amino acids of the amino acid sequence of the REIC/Dkk-3 protein as set forth in SEQ ID NO: 2. A nucleotide sequence of a DNA encoding the Cys-rich domain protein is set forth in SEQ ID NO: 3, and an amino acid sequence thereof is set forth in SEQ ID NO: 4. Besides, the partial protein obtained by deleting 120 amino acids at the N-terminal from the full-length REIC/Dkk-3 protein corresponds to a region consisting of an amino acid sequence from the 142th to the 350th amino acids of the amino acid sequence of the REIC/Dkk-3 protein set forth in SEQ ID NO: 2. A nucleotide sequence of a DNA encoding the partial protein obtained by deleting 120 amino acids at the N-terminal from the full-length REIC/Dkk-3 protein is set forth in SEQ ID NO: 5, and an amino acid sequence thereof is set forth in SEQ ID NO: 6.

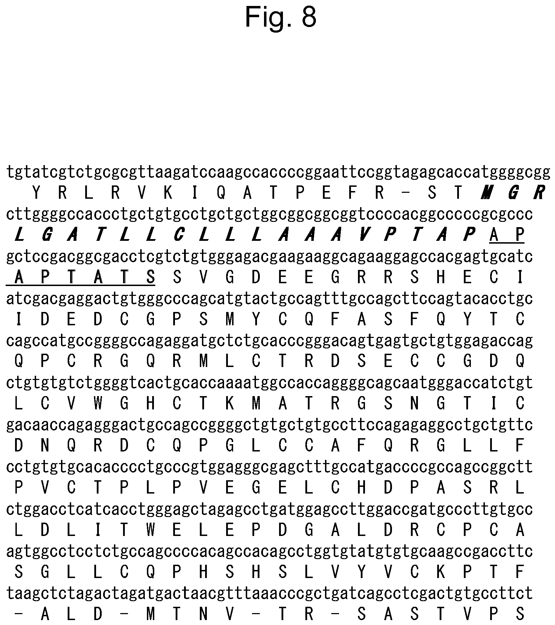

When the partial protein is to be actually produced as a recombinant protein, a protein is synthesized with the signal sequence at the N-terminal of the REIC/Dkk-3 linked, so as to be secreted from a host cell. At this point, since the protein is designed with several amino acids allowed to remain downstream from a portion of the secretion signal to be truncated by processing, amino acids on the C-terminal side of a signal peptide of the full-length REIC/Dkk-3 protein as set forth in SEQ ID NO: 2 are added to an N-terminal side of the partial protein to be produced. The protein to be added is, for example, APAPTATS (SEQ ID NO: 7). For example, Figure 8 illustrates a DNA sequence (SEQ ID NO: 8) and an amino acid sequence thereof (SEQ ID NO: 9) to be used in producing the Cys-rich domain protein as a recombinant protein. In Figure 8, an italic portion corresponds to the signal sequence, and underlined APAPTATS following it corresponds to the added sequence.

The REIC/Dkk-3 protein or the partial protein thereof of the present invention is a protein having the above-described amino acid sequence, namely, the amino acid sequence as set forth in SEQ ID NO: 2, SEQ ID NO: 4 or SEQ ID NO: 6, or an amino acid sequence substantially the same as the amino acid sequence, and having a TGFβ inhibitory effect. Here, substantially the same amino acid sequence can be an amino acid sequence obtained by replacing, deleting and/or adding one, a plurality of, or several (1 to 10, preferably 1 to 5, and further preferably 1 or 2) amino acids in the amino acid sequence, or one having sequence identity to the amino acid sequence, calculated by using BLAST (Basic Local Alignment Search Tool at the National Center for Biological Information) or the like (for example, using default, namely, initially set, parameters), of at least 85% or more, preferably 90% or more, further preferably 95% or more, still further preferably 97% or more, and particularly preferably 99% or more.

Besides, a DNA encoding the REIC/Dkk-3 protein of the present invention or the partial protein thereof can be a DNA having sequence identity to the nucleotide sequence as set forth in SEQ ID NO: 1, SEQ ID NO: 3 or SEQ ID NO: 5, calculated by using BLAST (Basic Local Alignment Search Tool at the National Center for Biological Information) or the like (for example, using default, namely, initially set, parameters), of at least 85% or more, preferably 90% or more, further preferably 95% or more, still further preferably 97% or more, and particularly preferably 99% or more, or one encoding a protein obtained by replacing, deleting and/or adding one, a plurality of, or several (1 to 10, preferably 1 to 5, and further preferably 1 or 2) amino acids in the amino acid sequence of the protein encoded by the DNA, and having the TGFβ inhibitory effect.

The REIC/Dkk-3 protein or the partial protein thereof can be obtained through chemical synthesis based on the above-described sequence information. Alternatively, it can be obtained as a recombinant protein by genetic engineering technique. Specifically, a DNA encoding a partial protein of the REIC/Dkk-3 protein of the present invention is introduced into an appropriate vector, the vector is inserted into a host, the resultant host is cultured, and a polypeptide may be obtained from the resultant culture. The vector used for inserting the DNA of the present invention is not especially limited as long as it can be replicated in a host, examples include a plasmid DNA and a phage DNA, and any known vector can be used. At this point, a eukaryotic cell or prokaryotic cell system can be used as the host. Examples of the eukaryotic cell include animal cells such as an established mammalian cell system of a human, a rodent or the like, an insect cell system, a filamentous fungus cell and a yeast cell, and an example of the prokaryotic cell includes a bacterial cell such as *E.coli.* cell. The REIC/Dkk-3 protein of the present invention or the partial protein thereof can be obtained from a culture by culturing a host cell containing a DNA encoding the REIC/Dkk-3 protein or the partial protein thereof *in vitro* or *in vivo* and culturing the resultant host by a known method. Here, the term "culture" means any of a culture supernatant, a cultured cell or a cultured bacteria, or a disrupted product of a cell or a bacteria. The protein thus expressed and produced can be purified from the culture. For the purification, any purification method usually employed for a protein may be employed, and the purification can be performed by, for example, appropriately selecting and combining ion-exchange chromatography, affinity chromatography, gel filtration, ultrafiltration, salting-out, dialysis and the like. Alternatively, the partial protein of the REIC/Dkk-3 protein of the present invention can be obtained in accordance with the description of WO01/038528.

The REIC/Dkk-3 DNA can be obtained from a human cell, a human tissue or the like based on the sequence information of SEQ ID NO: 1, 3 or 5. Alternatively, it can be obtained in accordance with the description of WO01/038528.

The present invention further embraces a vector containing the REIC/Dkk-3 DNA. When the vector is introduced into a subject, the REIC/Dkk-3 protein is expressed in a living body of the subject to work as the TGFβ inhibitor.

The introduction of a target gene (DNA) into a subject in the gene therapy can be performed by any known method. Examples of a method for introducing a gene into a subject include a method using a viral vector and a method using a non-viral vector, and various methods are known (Separate Volume of Experimental Medicine, Idenshi-chiryo no Kiso Gijutsu (Basic Technique of Gene Therapy), Yodosha Co., Ltd., 1996; Separate Volume of Experimental Medicine, Idenshi Donyu & Hatsugen Kaiseki Jikken-ho (Experimental Method for Gene Transfection & Expression Analysis), Yodosha Co., Ltd., 1997; Idenshi Chiryo Kaihatsu Kenkyu Handbook (Handbook of Development and Study of Gene Therapy), edited by Japan Society of Gene Therapy, NTS Inc., 1999).

A method in which a viral vector such as adenovirus, adeno-associated virus (AAV) or retrovirus is used as a viral vector for introducing a gene is representative. When a target gene is introduced into a DNA virus or an RNA virus such as detoxified retrovirus, herpes virus, vaccinia virus, poxvirus, poliovirus, sindbis virus, Sendai virus, SV40 or immunodeficiency virus (HIV), and a cell is infected with the resultant recombinant virus, the gene can be introduced into the cell.

When the gene of the present invention is used in the gene therapy using a virus, an adenoviral vector is preferably used. As characteristics of the adenoviral vector, (1) a gene can be introduced into various types of cells, (2) a gene can be efficiently introduced also into a cell in a growth arrest state, (3) condensation can be performed by centrifugation, and a high titer (of 10¹⁰ to 10¹¹ PFU/ml or more) virus can be obtained, and (4) it is suitably used for directly introducing a gene into a tissue cell *in vivo.* As the adenovirus for the gene therapy, a second generation adenoviral vector (Lieber, A. et al., J. Virol., 70, 8944, 1996; Mizuguchi, H. & Kay, M. A., Hum. Gene Ther., 10, 2013, 1999) obtained by deleting not only E1/E3 region but also E2 or E4 region from a first generation adenoviral vector lack of E1/E3 region (Miyake, S. et al., Proc. Natl. Acad. Sci. USA., 93, 1320, 1996), and a third generation (GUTLESS) adenoviral vector (Steinwaerder, D. S. et al., J. Virol. 73, 9303, 1999) substantially completely lack of adenovirus genome have been developed, and for introducing the gene of the present invention, the adenoviral vector is not especially limited but any of the adenoviral vectors can be used. Besides, when an adeno-AAV hybrid vector (Recchia, A. et al., Proc. Natl. Acad. Sci. USA., 96, 2615, 1999) obtained by imparting integration ability to chromosome of AAV, an adenoviral vector provided with integration ability to chromosome by using a gene of a transposon, or the like is used, the present technique can be applied to long-term gene expression. Besides, when a peptide sequence exhibiting tissue-specific migration is inserted into H1 loop of adenoviral fiber, tissue-specificity can be imparted to an adenoviral vector (Mizuguchi, H. & Hayakawa, T., Nippon Rinsho, 7, 1544, 2000).

In the present invention, an adenoviral vector containing the REIC/Dkk-3 DNA is referred to as Ad-REIC.

Alternatively, without using the above-described virus, a recombinant expression vector in which a gene expression vector such as a plasmid vector has been integrated can be used to introduce the target gene into a cell or a tissue. The gene can be introduced into a cell by, for example, lipofection transfection, calcium phosphate coprecipitation transfection, DEAE-dextran transfection, or direct DNA injection using a glass micro-tube. Alternatively, a recombinant expression vector can be incorporated into a cell by gene transfection through an internal liposome, gene transfection through an electrostatic type liposome, an HVJ-liposome transfection, improved HVJ-liposome transfection (HVJ-AVE liposome transfection), a method using an HVJ-E (envelope) vector, receptor-mediated gene transfection, a method in which a DNA molecule is transfected into a cell together with a carrier (a metal particle) by a particle gun, naked-DNA direct transfection, or transfection using various polymers. The expression vector used in such a case can be any expression vector as long as the target gene can be expressed in a living body, and examples of the expression vector include pCAGGS (Gene 108, 193-200 (1991)), pBK-CMV, pcDNA3, 1, pZeoSV (Invitrogen, Stratagene) and pVAX1.

The vector containing the REIC/Dkk-3 DNA may appropriately contain a promoter or an enhancer for transferring the gene, poly A signal, a marker gene for labeling and/or selecting a cell in which the gene has been transfected, and the like. As the promoter used here, any known promoter can be used.

For introducing the vector containing the REIC/Dkk-3 DNA of the present invention into a subject, an *in vivo* method in which a gene therapeutic agent is directly introduced into a body, an *ex vivo* method in which a type of cell is taken out of a human body, a gene therapeutic agent is introduced into the cell outside the body, and the resultant cell is returned to the body, or the like may be employed (Nikkei Science, 1994, April issue, pp. 20-45; The Pharmaceuticals monthly, 36(1), 23-48 (1994); Extra Edition of Experimental Medicine, 12(15), 1994; Idenshi Chiryo Kaihatsu Kenkyu Handbook (Handbook of Development and Study of Gene Therapy), edited by Japan Society of Gene Therapy, NTS Inc., 1999).

The REIC/Dkk-3 protein or the partial protein thereof binds to a TGFβ receptor to inhibit the TGFβ from binding to the TGFβ receptor. As a result, the signaling from the TGFβ is inhibited.

The TGFβ is a multifunctional cytokine involved in growth and differentiation of a cell, apoptosis, blood formation, bone development, extracellular matrix formation, an immune reaction, an inflammatory reaction, fibrosis of an organ or a tissue, and the like. The TGFβ is involved, through its functions, in pathogenesis of various diseases such as cancer, fibrosis, nervous diseases, rheumatism, allergic diseases and arteriosclerosis. Besides, in a tumor local site, the TGFβ is secreted from a cancer cell to induce a regulatory T cell (Treg cell) and induce immunosuppression. As a result, attack of an immune cell against a cancer cell is inhibited. The REIC/Dkk-3 protein inhibits the TGFβ from binding to a TGFβ receptor, and as a result, a signaling pathway from the TGFβ for inducing the immunosuppression is inhibited to reduce an inhibitory immune cell in the tumor local site and restore tumor immune response, and thus, the immunosuppression is released to recover and activate the attack of an immune cell against a cancer cell. In other words, the REIC/Dkk-3 protein mainly affects an immune system cell to inhibit a reaction between the TGFβ and its receptor in the immune system cell.

The REIC/Dkk-3 protein affects a cell of an immune system such as a T lymphocyte-like cell, and does not affect another cell such as a fibroblast.

The REIC/Dkk-3 protein cannot completely interrupt the activity of the TGFβ even in a high concentration region, but exhibits an inhibitory effect selectively to an immune system. In other words, the REIC protein can reduce the TGFβ activity selectively to an immune system, and can be used as an immunomodulator for gently activating anti-tumor immune activity in a tumor local site. There is a fear of an adverse reaction in a drug strongly inhibiting the TGFβ involved in various physiological functions, but the REIC can reduce the TGFβ activity with low affinity and specifically to an immune system, and therefore, with reducing an adverse reaction otherwise caused by inhibiting the TGFβ, an activated state of the tumor immune response ideal for cancer treatment can be induced.

The TGFβ receptor to which the REIC/Dkk-3 protein binds includes both a type I receptor (TGFβ RI) and a type II receptor (TGFβ RII).

The TGFβ inhibitor comprising, as an active ingredient, the REIC/Dkk-3 protein or the partial protein thereof, or a DNA encoding any of these proteins can be used for prevention or treatment of a TGFβ-related disease. Here, the TGFβ-related disease refer to a disease caused by abnormalities of the TGFβ signaling, and the TGFβ inhibitor comprising, as an active ingredient, the REIC/Dkk-3 protein or the partial protein thereof, or a DNA encoding any of these proteins normalizes the TGFβ signaling by inhibiting the TGFβ from binding to the TGFβ receptor, and thus, the TGFβ-related disease can be prevented or treated.

An example of the TGFβ-related disease includes cancer.

Examples of the cancer to be prevented or treated by the TGFβ inhibitor of the present invention include solid cancers such as brain/nerve tumor, skin cancer, stomach cancer, lung cancer, liver cancer, lymphoma/leukemia, colon cancer, pancreatic cancer, anal/rectal cancer, esophageal cancer, uterine cancer, breast cancer, adrenal cancer, kidney cancer, renal pelvis and ureter cancer, bladder cancer, prostate cancer, ureter cancer, penile cancer, testicular cancer, osteoma/osteosarcoma, multiple myeloma, leiomyoma, rhabdomyoma and mesothelioma, and blood cancer.

A preventing or treating agent of the present invention comprises the REIC/Dkk-3 protein or the partial protein thereof, or the DNA encoding any of these proteins, and a pharmacologically acceptable carrier, diluent or excipient. The agent for preventing or treating a TGFβ-related disease can be administered in various forms, and examples include oral administration in the form of a tablet, a capsule, a granule, a powder, a syrup or the like, and parenteral administration in the form of an injection (including subcutaneous injection, intravenous injection, intramuscular injection and intraperitoneal injection), a drop, a suppository, a spray, an eye drop, a transnasal agent, a transdermal agent, a transmucosal agent, a transpulmonary agent and a patch.

The preventing or treating agent of the present invention may be systemically administered by injection or the like, or can be locally administered. For example, when administered to a cancer site by injection, its effects can be exhibited.

The preventing or treating agent of the present invention contains a carrier, a diluent or an excipient usually used in the pharmaceutical field. For example, as a carrier or an excipient for a tablet, lactose, magnesium stearate or the like is used. As an aqueous liquid for an injection, a saline, an isotonic liquid containing glucose or another adjuvant, or the like is used, and an appropriate dissolution assisting agent, for example, alcohol, polyalcohol such as propylene glycol, a nonionic surfactant or the like may be used together. As an oily liquid, sesame oil, soybean oil or the like is used, and benzyl benzoate, benzyl alcohol or the like may be used together as a dissolution assisting agent.

A dose thereof is varied depending on the symptom, the age, the weight and the like, and a dose of 0.001 mg to 100 mg may be administered by subcutaneous injection, intramuscular injection or vascular injection once every several days, several weeks or several months. Alternatively, when the vector containing the REIC/Dkk-3 DNA is used, for example, the vector corresponding to a dose of 10⁷ to 10⁹ pfu (plaque forming units) may be administered.

Through examination performed *in vitro,* the reaction between the TGFβ and the receptor thereof is inhibited in a concentration-dependent manner in a concentration region of the REIC/Dkk-3 protein of 40 nM or more, and preferably 50 nM or more. Accordingly, it may be administered in an amount with which the concentration in blood after the administration can be 40 nM or more, and preferably 50 nM or more. Incidentally, even when the REIC/Dkk-3 protein is administered at a high dose of, for example, 375 nM or more, the reaction between the TGFβ and the receptor thereof cannot be completely inhibited, and therefore, an adverse reaction otherwise caused by strongly inhibiting the TGFβ can be reduced.

The REIC/Dkk-3 protein has not been observed for an adverse reaction such as toxicity, and the administration of a large dose of the REIC/Dkk-3 protein is expected to be highly safe.

The preventing or treating agent of the present invention exhibits a preventing and treating effect on a TGFβ-related disease, particularly cancer, even when singly administered. Besides, when the agent is used for prevention or treatment of cancer, it can be singly used as an immune checkpoint inhibitor, or can be used in combination with another immune checkpoint inhibitor. When the present agent and another immune checkpoint inhibitor are used in combination, the anticancer action is doubly induced, and hence a strong preventing or treating effect can be expected. In particular, when used in combination with an existing immune checkpoint inhibitor, a robust immune checkpoint in a tumor local site can be more likely to be released, and hence a response rate in the cancer immunotherapy with enhanced QOL can be more likely to be largely improved. As the administration method, either the gene therapy in which the REIC protein is forcedly expressed or the administration of a REIC protein preparation can be employed. Examples of the immune checkpoint inhibitor include an anti-PD-1 antibody, an anti-PD-L1 antibody, an anti-CTLA-4 antibody and an anti-CCR4 antibody.

### Examples

The present invention will now be specifically described with reference to the following examples, and it is noted that the present invention is not limited to these examples.

### 1. Search for Protein Binding to REIC Protein

A full-length REIC protein was produced in a secretion expression system using FreeStyle 293 cell as a host, and the resultant was separated and purified by ion exchange chromatography to attain high purity. The thus obtained sample was mixed with NHS Mag Sepharose (GE Healthcare) to prepare a REIC-coated magnetic bead. Next, GM-CSF (10 ng/mL) and IL-4 (10 ng/mL) were added to commercially available CD14-positive human peripheral blood monocyte (Lonza) together, and the resultant was induced to differentiate into a dendritic cell-like cell and cultured for 6 days. The thus obtained culture supernatant was mixed with the REIC-coated magnetic bead at ambinet temperature to collect a molecule binding to the bead. The molecule binding to the magnetic bead was eluted with glycine/hydrochloric acid (pH 2.9), and a fraction containing a REIC-binding protein was collected. The thus collected sample was reacted with a protease: trypsin, and the resultant digestion fragment was analyzed by HPLC/QTOF mass analysis to identify a protein contained in the sample from MASCOT database. Although a component exhibiting a remarkable bond was not listed, it was confirmed that an extracellular domain of the TGFβ type I receptor (TGFβRI-ECD) was included in a list of candidate molecules in which a partial fragment was detected.

### 2. Search for Protein Binding to REIC Protein

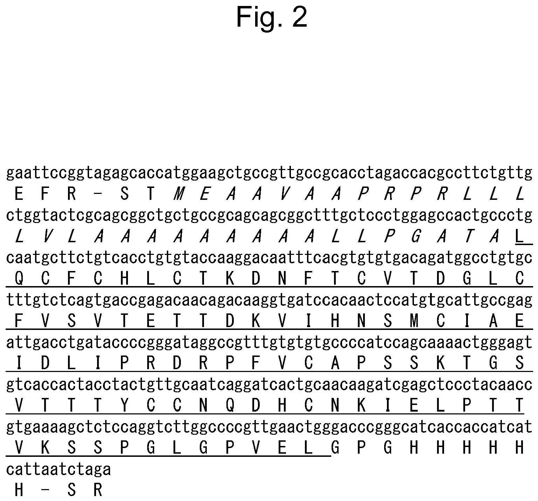

In order to experimentally prove that the REIC protein and the TGFβRI-ECD bind to each other, expression plasmid DNAs of secretory proteins of these were prepared. As the REIC protein, three proteins of FL-REIC(SGE-REIC) secretory-expressing the full-length REIC, REIC C domain (SGE-REIC-C) in which 120 amino acids at the N-terminal had been deleted, and REIC-17kDa (SGE-REIC-17kDa) corresponding to the Cys-rich domain alone were used. On the other hand, as the TGFβRI-ECD protein, two proteins of TGFβRI-ECD (SGE-TGFβRI-ECD) and sigREIC-TGFβRI-ECD (SGE-sigREIC-TGFβRI-ECD) in which a REIC secretion signal had been added in expectation of expression level improvement were used. Figure 2 illustrates a DNA sequence (SEQ ID NO: 10) of an artificial gene of human TGFβRI-ECD (Uniprot: P36897) used in the synthesis of the TGFβRI-ECD protein, and an amino acid sequence (following a signal sequence, SEQ ID NO: 11) thereof. In the amino acid sequence, an italic portion corresponds to the secretion signal, and an underlined portion corresponds to a TGFβRI-ECD portion. At the C-terminal, His Tag (HHHHHH) is added.

### 3. Binding Experiment in Co-expression System of REIC Protein and TGFβ-RI

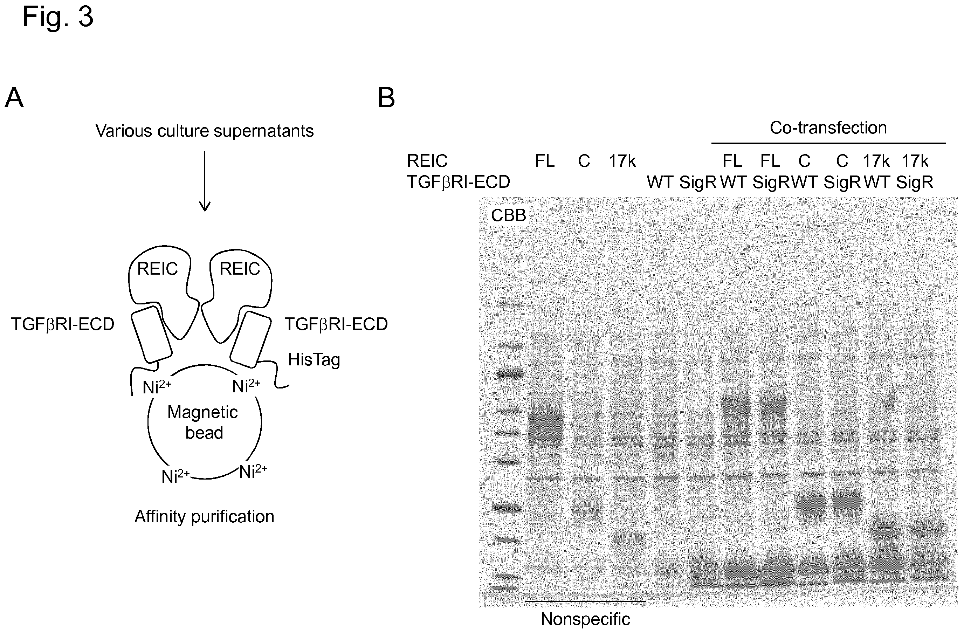

Various REIC and TGFβ-RI expression plasmid DNAs were used to perform transient gene transfection by lipofection transfection using a transfection reagent: 293 Fectin on FreeStyle 293F cells, the resultant cells were shaking cultured at 37°C for 3 days, and the thus obtained culture supernatants were collected. To 1 mL of each of the culture supernatants, 50 µL of His Mag Sepharose Ni (GE Healthcare: magnetic bead) washed with X1 PBS was added, and the resultant was gently mixed at room temperature for 1 hour. Thereafter, the bead was collected with a magnet, the resultant was washed with X1 PBS three times, 40 µL of X1 SDS sample buffer (containing a reducing agent) was added to the resultant to extract a protein specifically binding to the magnetic bead, and the protein interactive with TGFβRI-ECD was analyzed by SDS-PAGE (Figure 3). As a result, it was confirmed that when the TGFβRI-ECD is co-expressed with various REIC, a complex having a band strength of about 1:1 was concentrated on a Ni²⁺-Affinity magnetic bead, and thus, it was confirmed that these form a complex together. Besides, it was confirmed in this case that the REIC protein may be the REIC 17kDa that is the minimal domain responsibility for the immune activity.

### 4. Binding Experiment in Co-expression System of REIC protein, TGFβRI-Fc and TFGβRII-Fc

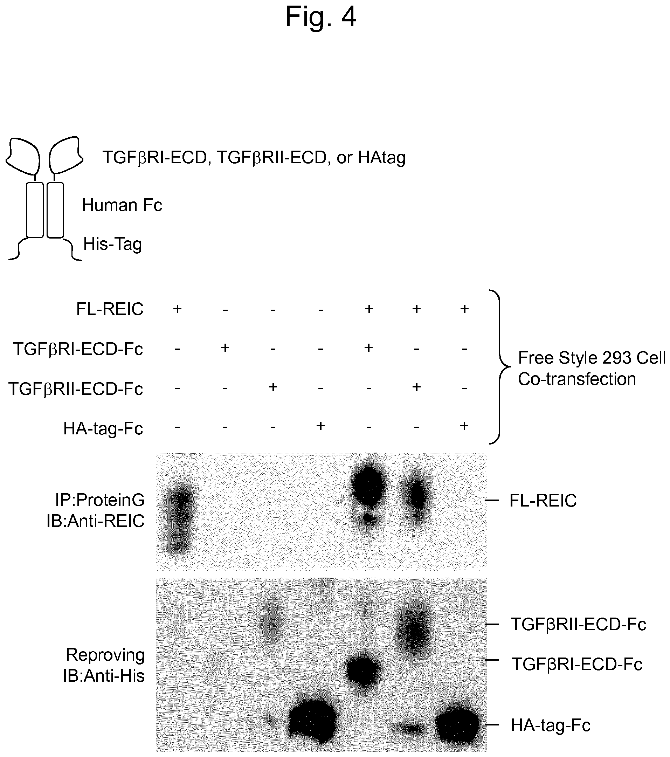

It was found through various experiments that the TGFβRI-ECD has a problem of low stability, and for purposes of improvement, a protein production system in which TGFβRI-ECD was fused on the N-terminal side of the Fc region of human immunoglobulin G (IgG) was constructed. Simultaneously, expression plasmid DNA vectors of Fc fusion proteins in which TGFβRII-ECD (Uniprot: P37173) and HAtag were respectively added were produced. Each of these genes was combined with an expression vector of FL-REIC to be co-transfected into FreeStyle 293 cell, Fc fusion protein was collected by using Protein G-coated magnetic bead (Protein G-Mag Sepharose, GE Healthcare) binding to the Fc region, and it was confirmed, by a Western blotting method using an anti-REIC antibody, that a complex was formed together with the REIC protein in a culture supernatant (Figure 4). As a result, it was confirmed that the REIC protein binds not only to the TGFβRI-ECD but also to the TGFβRII-ECD. On the other hand, the Fc protein in which Ha-tag had been added did not bind to REIC, and therefore, it was confirmed that the TGFβRI-ECD and the TGFβRII-ECD respectively specifically bind to the REIC protein.

### 5. Binding Experiment of REIC protein to TGFβRI-Fc and TFGβRII-Fc (ELISA)

Each of TGFβRI-ECD-Fc and TGFβRII-ECD-Fc proteins was secretory-expressed/purified as a recombinant protein, the resultant was added by 100 µL onto a 96-well ELISA plate in a concentration of 2.5 µg/mL, and the resultant was allowed to stand overnight at 4°C for immobilization. The wells were washed with 100 µL of X1 PBS three times, 200 µL of Blocking One (Nacalai Tesque Inc.) was added to each well, and the resultant was allowed to stand at room temperature for 1 hour. The wells were washed with 100 µL of X1 PBS-T three times, and then, full-length REIC diluted with X1 PBS-T + 0.1% BSA having been subjected to serial dilution from 5µM to 0.32 nM by X1/5 was added by 100 µL, and the resultant was allowed to stand at 4°C overnight.

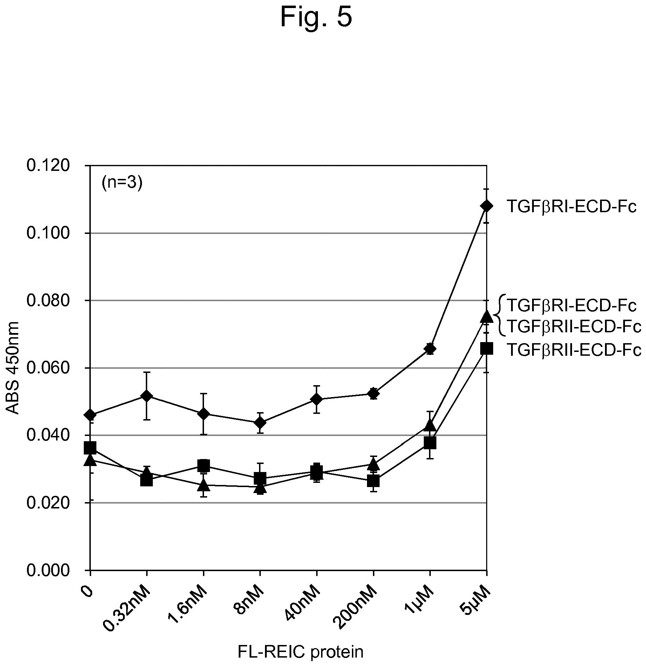

The wells were washed with 100 µL of X1 PBS-T three times, a 1000-fold diluted biotinylated REIC polyclonal antibody was added to each well by 100 µL, and the resultant was allowed to stand at room temperature for 1 hour. The wells were washed with 100 µL PBS-T1 three times, and 5000-folded diluted Streptavidin-HRP was added to each well by 100 µL, and the resultant was allowed to stand at room temperature for 1 hour. The wells were washed with 100 µL of X1 PBS-T three times, a coloring solution was added to each well by 100 µL, a coloring reaction was stopped by adding 50 µL of a stop solution to each well when appropriately colored, and the resultant was subjected to colorimetric analysis at a wavelength of 450 nm (Figure 5). As a result, it was found that the binding affinity between the TGFβRI-ECD and REIC is rather higher than that of the TGFβRII-ECD, and thus, results having the same tendency as in the Co-transfection experiment (Figure 4) were obtained. In this binding experiment, however, the binding was found at 1 µM or more, and hence it was confirmed that the binding affinity is not very high.

### 6. Verification of TGFP Inhibitory Ability by REIC Protein

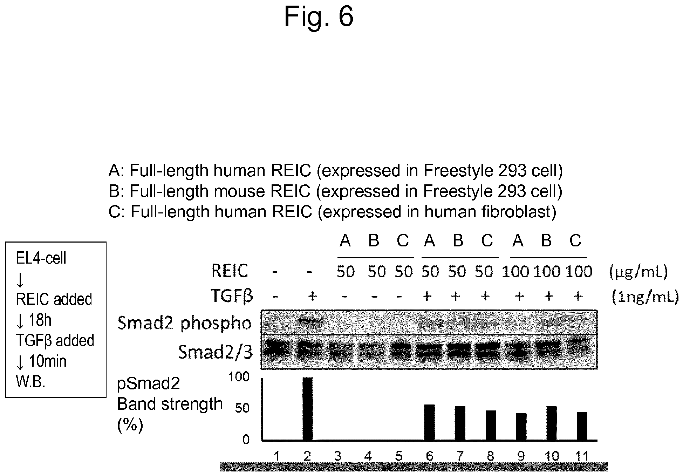

In a mouse T lymphocyte-derived EL4 cell, phosphorylation of intracellular Smad 2 is induced through stimulation with the TGFβ. The EL4 cell is also known as a model system capable of inducing regulatory T cell (Treg)-like differentiation (Nat. Immunol. 9, 194, 2007), and influence of the REIC protein on intracellular signaling of the TGFβ to the EL4 cell was checked. A TGFβ protein (Wako Pure Chemical Industries Ltd.) dissolved in a concentration of 20 µg/mL in a 4mM HCl + 0.1% HSA was used. The EL4 cell was cultured in an IMDM medium (Gibco) supplemented with 10% FBS using a 6-well plate. As the REIC protein to be added to the cultured EL4 cell, three proteins of human and mouse FL-REIC produced by secretion in Free Style 293 cell, and human FL-REIC prepared by using a production system using a human normal fibroblast glycosylated with a larger molecule were used, and each of these was added to a culture supernatant of the EL4 cell to perform incubation overnight at 37°C. The TGFβ was added to each well after the treatment with the REIC, and the resultant was allowed to stand at 37°C for 10 minutes. Thereafter, the supernatant was gently removed, 100 µL of Lysis Buffer (20 mM Hepes, pH 7.5, 500 mM NaCl, 1% NP-40, protease inhibitor (Nacalai Tesque Inc.), phosphatase inhibitor (Nacalai Tesque Inc.)) was added to each well, and the cell was collected. After the resultant cell lysate was subjected to sonication treatment (using Bioruptor), the resultant was subjected to centrifugation at 13,000 rpm at 4°C for 10 minutes to collect a supernatant. The supernatant was separated by SDS-PAGE, and then transferred to a nitrocellulose membrane. The resultant membrane was treated with a blocking agent of 5% nonfat dry milk, and was then subjected to the Western blotting performed using an anti-Smad 2 phospho antibody (Cell Signaling, 1000-fold diluted). Besides, the same membrane was subjected to re-probing treatment to verify each Smad 2/3 protein by using an anti-Smad 2/3 antibody (Cell Signaling, 2000-fold diluted) (Figure 6). Figure 6 illustrates a Western blotting image and the intensity of phosphorylated Smad 2 band (a graph in a lower portion). It was confirmed based on these results that the signaling within the EL4 cell caused by the stimulation with the TGFβ is reduced by adding the REIC protein.

### 7. Competitive Inhibition by REIC Protein of Cell Growth Inhibitory Activity induced by TGFβ

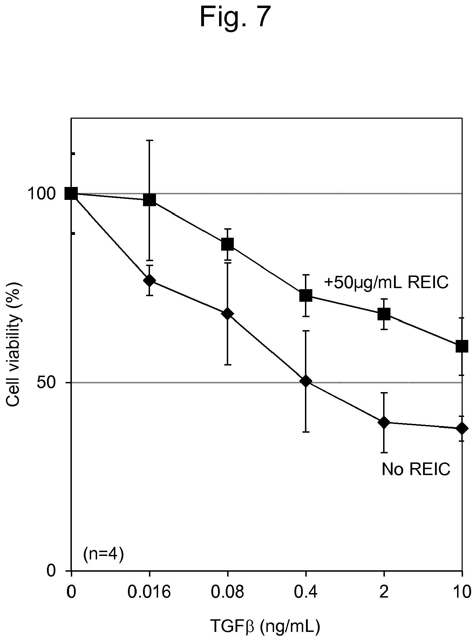

In order to verify, in a bioassay system, that the REIC protein reduces the activity of the TGFβ, an effect on human erythroleukemia cell line: TF-1 cell was evaluated. The TF-1 cell is a cultured cell growing proliferate cytokine-dependently, and it is known that growth inhibitory activity is found when the TGFβ, that is, inhibitory cytokine, is added. In this evaluation experiment, the TF-1 cell was cultured in an RPMI medium supplemented with 10% FBS and GM-CSF (2 ng/mL), the resultant TF-1 cell was washed twice with an RPMI medium supplemented with 10% FBS, 100µL of the resultant TF-1 cell was put in each well of a 96-well plate in a concentration of 1.0 x 10⁵ cells/mL, and IL-5 was added thereto in a concentration of 4 ng/mL. Besides, human FL-REIC protein and the TGFβ were added to each well, and the resultant was cultured at 37°C for 2 days. With respect to the cell growth in each well, the colorimetric analysis was performed using Cell Counting Kit-8 (Dojindo Laboratories) to calculate cell growth activity (Figure 7). As a result, it could be confirmed that the TGFβ activity of the immunosuppressive factor is reduced when the REIC protein coexists. It was verified, in this assay, as a gentle competitive effect, and similar effects were successfully obtained with high reproducibility through the experiment performed three times.

### 8. Verification of TGFβ Inhibitory Ability by REIC Protein

Each cell (EL-4, human fibroblast) was seeded in a 6-well plate, a recombinant REIC protein was added to a medium, and the resultant was allowed to stand at 37°C for 18 hours.

To a well for adding the TGFβ, the TGFβ was added to a final concentration of 1 ng/mL, and the resultant was allowed to stand at 37°C for 10 minutes. After removing the medium of the 6-well plate with an aspirator, the resultant cell was dissolved in each well in 100 µL of Cell Lysis buffer (20 mM Hepes, pH 7.5, 500 mM NaCl, 1% NP-40) containing Protease inhibitor cocktail (Nacalai Tesque Inc.) and Phosphatase inhibitor cocktail (Nacalai Tesque Inc.). The resultant cell was collected using a cell lifter and transferred to a 1.5 mL tube, and subjected to sonication treatment using Bioruptor to prepare a cell lysate. The thus obtained cell lysate was subjected to centrifugation at 4°C and 13,000 rpm for 10 minutes, and a supernatant was put in a fresh tube. 20 µL each of the supernatant was analyzed by the Western blotting using, as a primary antibody, Anti-Smad 2/3 and Anti-Smad 2/3 phospho-specific antibody (Cell Signaling).

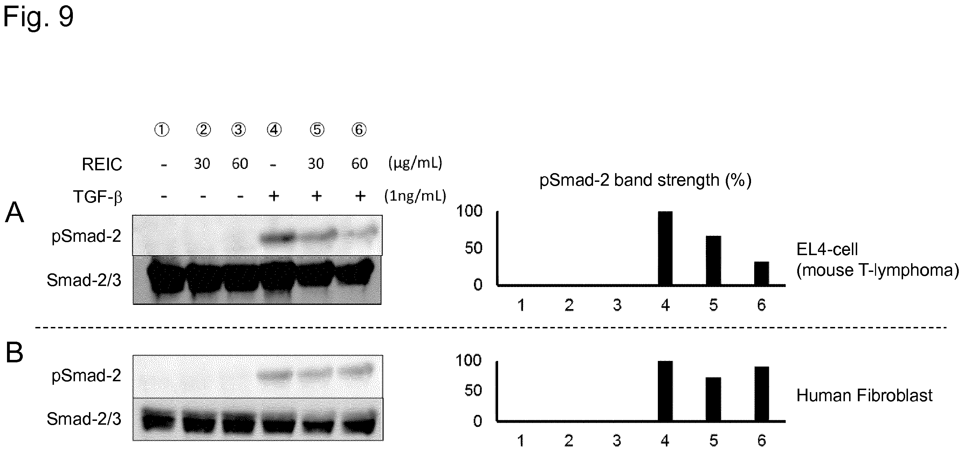

The results are illustrated in Figure 9. Figure 9A illustrates the results obtained from the EL4 cell and Figure 9B illustrates the results obtained from the human fibroblast. Figure 9 illustrates a Western blotting image and the intensity of phosphorylated Smad 2 band (a graph on the right side).

It was revealed based on the results of Figure 9 that the REIC protein exhibits TGFβ activity inhibitory effect selectively to an immune system.

### 9. Inhibitory Effect on TGFβ Pathway of REIC Protein (Effect derived from Reporter Gene)

### (1) Examination using EL-4 Cell

A plasmid expressing a luciferase reporter in accordance with stimulation with the TGFβ, pGL4.48 (Promega) was transfected into various cells to perform luciferase assay. First, the EL4 cell was cultured in a 96-well plate to less than 70% confluence. A medium supplemented with serum was removed with an aspirator, and the medium was replaced with Opti-MEM. In accordance with a manual attached to a transfection reagent, Lipofectamine 3000 (Invitrogen), 100 ng of pGL4.48 DNA was transfected into each well. The resultant was incubated at 37°C in the presence of 5% CO₂ for 3 hours, a recombinant REIC protein was added to a final concentration of 50 µg/mL or 100 µg/mL, and the resultant was incubated at 37°C in the presence of 5% CO₂ for 18 hours. Thereafter, the TGFβ was added thereto to a final concentration of 10 µg/mL, and the resultant was incubated at 37°C in the presence of 5% CO₂ for 3 hours. An expression level of a reporter gene in the cell was quantitatively evaluated using Steady-Glo Luciferase Assay System (Promega) and a luminometer.

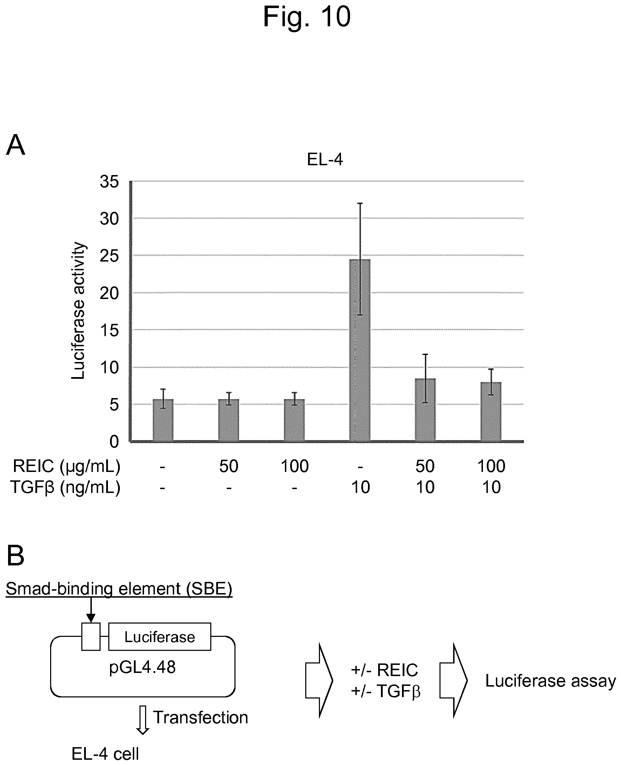

Figure 10 illustrates the outline of this assay (Figure 10B) and results obtained from the EL-4 cell (Figure 10A). The ordinate of Figure 10A corresponds to luciferase activity.

### (2) Examination of REIC Protein Concentration

Under the same conditions as in (1) above, a TGFβ pathway inhibitory effect was checked with the added concentration of the REIC protein changed from 0 to 3000 nM.

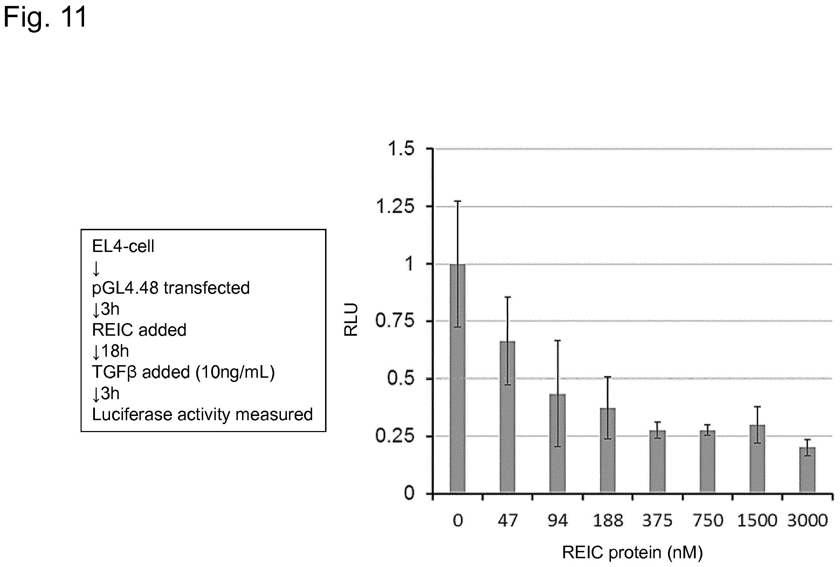

Figure 11 illustrates the outline of this method and results. The ordinate corresponds to relative light unit (RLU) obtained on the assumption that a case not containing the REIC protein is 1.

It was found, based on the results of Figures 10 and 11, that the REIC protein cannot completely inhibit the activity of the TGFβ even in a high concentration region thereof, but exhibits an inhibitory effect selectively to an immune system. In other words, the REIC protein can reduce the TGFβ activity selectively to an immune system, and can be used as an immunomodulator for gently activating the anti-tumor immune activity in a tumor local site.

### Industrial Applicability

REIC/Dkk-3 protein or a partial protein thereof, or a DNA encoding any of these proteins can be used for prevention or treatment of a TGFβ-related disease.

### Sequence Listing Free Text

### SEQ ID NOS: 7 to 11 Synthesis

All publications, patents, and patent applications herein mentioned are herein incorporated in their entirety by reference into the specification.

## Claims

1. A TGFβ inhibitor, comprising, as an active ingredient, REIC/Dkk-3 protein or a partial protein thereof comprising a Cys-rich domain thereof, or a DNA encoding any of the proteins.

2. The TGFβ inhibitor according to claim 1, comprising the REIC/Dkk-3 protein or the partial protein thereof comprising the Cys-rich domain thereof, the TGFβ inhibitor inhibiting a reaction between TGFβ and a receptor of the TGFβ concentration-dependently in a concentration region corresponding to an active dose of 50 nM or more.

3. The TGFβ inhibitor according to claim 1 or 2, the TGFβ inhibitor not completely inhibiting a reaction between TGFβ and a receptor of the TGFβ even when an active dose is high.

4. The TGFβ inhibitor according to claim 3, wherein the TGFβ inhibitor does not completely inhibit the reaction between the TGFβ and the receptor even when the active dose is 375 nM or more.

5. An anti-tumor immune activating agent, comprising the TGFβ inhibitor according to any one of claims 1 to 4.

6. An agent for preventing or treating a TGFβ-related disease, comprising the TGFβ inhibitor according to any one of claims 1 to 4.

7. The agent for preventing or treating a TGFβ-related disease according to claim 6, the agent affecting an immune system cell to inhibit a reaction between TGFβ and a receptor of the TGFβ in the immune system cell.

8. The agent for preventing or treating a TGFβ-related disease according to claim 6 or 7, reduce adverse reaction otherwise caused by inhibition of TGFβ by not completely inhibiting a reaction between TGFβ and a receptor of the TGFβ.

9. The agent for preventing or treating a TGFβ-related disease according to any one of claims 6 to 8, the agent inhibiting a reaction between TGFβ and a receptor of the TGFβ concentration-dependently at a concentration corresponding to an active dose of 50 nM or more, and not completely inhibiting the reaction between the TGFβ and the receptor even at a high concentration of 375 nM or more.

10. The agent for preventing or treating a TGFβ-related disease according to any one of claims 6 to 9, wherein the TGFβ-related disease is cancer, and the agent activates anti-tumor immunity.

11. The agent for preventing or treating a TGFβ-related disease according to claim 10, wherein the agent releases immunosuppression which mediated by a regulatory T cell induced by TGFβ secreted from a cancer cell, and the agent restores and activates attack of an immune cell against a cancer cell.


SEQUENCE LISTING

<110>  Momotaro-Gene Inc.

<120>  A TGF beta inhibitor comprising REIC/Dkk3 protein as an
       active ingredient

<130>  PH-7013-PCT

<150>  JP 2016-121187
<151>  2016-06-17

<160>  11

<170>  PatentIn version 3.4

<210>  1
<211>  1053
<212>  DNA
<213>  Homo sapiens

<220>
<221>  CDS
<222>  (1)..(1053)

<400>  1
atg cag cgg ctt ggg gcc acc ctg ctg tgc ctg cta ctg gcg gcg gcg      48
gtc ccc acg gcc ccc gcg ccc gct ccg acg gcg acc tcg gct cca gtc      96
aag ccc ggc ccg gct ctc agc tac ccg cag gag gag gcc acc ctc aat      144
gag atg ttc cgc gag gtt gag gaa ctg gtg gag gac acg cag cac aaa      192
ttg cgc agc gcg gtg gaa gag atg gag gca gaa gaa gct gct gct aaa      240
gca tca tca gaa gtg aac ctg gca aac tta cct ccc agc tat cac aat      288
gag acc aac aca gac acg aag gtt gga aat aat acc atc cat gtg cac      336
cga gaa att cac aag ata acc aac aac cag gct cga caa atg gtc ttt      384
tca gag aca gtt atc aca tct gtg gga gac gaa gaa ggc aga agg agc      432
cac gag tgc atc atc gac gag gac tgt ggg ccc agc atg tac tgc cag 480
ttt gcc agc ttc cag tac acc tgc cag cca tgc cgg ggc cag agg atg 528
ctc tgc acc cgg gac agt gag tgc tgt gga gac cag ctg tgt gtc tgg 576
ggt cac tgc acc aaa atg gcc acc agg ggc agc aat ggg acc atc tgt 624
gac aac cag agg gac tgc cag ccg ggg ctg tgc tgt gcc ttc cag aga 672
ggc ctg ctg ttc cct gtg tgc ata ccc ctg ccc gtg gag ggc gag ctt 720
tgc cat gac ccc gcc agc cgg ctt ctg gac ctc atc acc tgg gag cta 768
gag cct gat gga gcc ttg gac cga tgc cct tgt gcc agt ggc ctc ctc 816
tgc cag ccc cac agc cac agc ctg gtg tat gtg tgc aag ccg acc ttc 864
gtg ggg agc cgt gac caa gat ggg gag atc ctg ctg ccc aga gag gtc 912
ccc gat gag tat gaa gtt ggc agc ttc atg gag gag gtg cgc cag gag 960
ctg gag gac ctg gag agg agc ctg act gaa gag atg gcg ctg ggg gag 1008
cct gcg gct gcc gcc gct gca ctg ctg gga ggg gaa gag att tag 1053

<210> 2
<211> 350
<212> PRT
<213> Homo sapiens

<400> 2

<210>  3
<211>  462
<212>  DNA
<213>  Homo sapiens

<220>
<221>  CDS
<222>  (1)..(462)

<400>  3
tct gtg gga gac gaa gaa ggc aga agg agc cac gag tgc atc atc gac        48
Ser Val Gly Asp Glu Glu Gly Arg Arg Ser His Glu Cys Ile Ile Asp
1               5                   10                  15

gag gac tgt ggg ccc agc atg tac tgc cag ttt gcc agc ttc cag tac        96
Glu Asp Cys Gly Pro Ser Met Tyr Cys Gln Phe Ala Ser Phe Gln Tyr
            20                  25                  30

acc tgc cag cca tgc cgg ggc cag agg atg ctc tgc acc cgg gac agt       144
Thr Cys Gln Pro Cys Arg Gly Gln Arg Met Leu Cys Thr Arg Asp Ser
        35                  40                  45

gag tgc tgt gga gac cag ctg tgt gtc tgg ggt cac tgc acc aaa atg       192
Glu Cys Cys Gly Asp Gln Leu Cys Val Trp Gly His Cys Thr Lys Met
    50                  55                  60

gcc acc agg ggc agc aat ggg acc atc tgt gac aac cag agg gac tgc       240
Ala Thr Arg Gly Ser Asn Gly Thr Ile Cys Asp Asn Gln Arg Asp Cys
65                  70                  75                  80

cag ccg ggg ctg tgc tgt gcc ttc cag aga ggc ctg ctg ttc cct gtg       288
Gln Pro Gly Leu Cys Cys Ala Phe Gln Arg Gly Leu Leu Phe Pro Val
                85                  90                  95

tgc ata ccc ctg ccc gtg gag ggc gag ctt tgc cat gac ccc gcc agc       336
Cys Ile Pro Leu Pro Val Glu Gly Glu Leu Cys His Asp Pro Ala Ser
            100                 105                 110

cgg ctt ctg gac ctc atc acc tgg gag cta gag cct gat gga gcc ttg       384
Arg Leu Leu Asp Leu Ile Thr Trp Glu Leu Glu Pro Asp Gly Ala Leu

115                 120                 125

gac cga tgc cct tgt gcc agt ggc ctc ctc tgc cag ccc cac agc cac        432
Asp Arg Cys Pro Cys Ala Ser Gly Leu Leu Cys Gln Pro His Ser His
    130                 135                 140

agc ctg gtg tat gtg tgc aag ccg acc ttc                                462
Ser Leu Val Tyr Val Cys Lys Pro Thr Phe
145                 150

<210>  4
<211>  154
<212>  PRT
<213>  Homo sapiens

<400>  4

Ser Val Gly Asp Glu Glu Gly Arg Arg Ser His Glu Cys Ile Ile Asp
1               5                   10                  15

Glu Asp Cys Gly Pro Ser Met Tyr Cys Gln Phe Ala Ser Phe Gln Tyr
            20                  25                  30

Thr Cys Gln Pro Cys Arg Gly Gln Arg Met Leu Cys Thr Arg Asp Ser
        35                  40                  45

Glu Cys Cys Gly Asp Gln Leu Cys Val Trp Gly His Cys Thr Lys Met
    50                  55                  60

Ala Thr Arg Gly Ser Asn Gly Thr Ile Cys Asp Asn Gln Arg Asp Cys
65                  70                  75                  80

Gln Pro Gly Leu Cys Cys Ala Phe Gln Arg Gly Leu Leu Phe Pro Val
                85                  90                  95

Cys Ile Pro Leu Pro Val Glu Gly Glu Leu Cys His Asp Pro Ala Ser
            100                 105                 110

Arg Leu Leu Asp Leu Ile Thr Trp Glu Leu Glu Pro Asp Gly Ala Leu
        115                 120                 125

Asp Arg Cys Pro Cys Ala Ser Gly Leu Leu Cys Gln Pro His Ser His
    130                 135                 140

Ser Leu Val Tyr Val Cys Lys Pro Thr Phe
145                 150

<210>  5
<211>  627
<212>  DNA
<213>  Homo sapiens

<220>
<221>  CDS
<222>  (1)..(627)

<400>  5
aga agg agc cac gag tgc atc atc gac gag gac tgt ggg ccc agc atg      48
Arg Arg Ser His Glu Cys Ile Ile Asp Glu Asp Cys Gly Pro Ser Met
1               5                   10                  15

tac tgc cag ttt gcc agc ttc cag tac acc tgc cag cca tgc cgg ggc      96
Tyr Cys Gln Phe Ala Ser Phe Gln Tyr Thr Cys Gln Pro Cys Arg Gly
            20                  25                  30

cag agg atg ctc tgc acc cgg gac agt gag tgc tgt gga gac cag ctg      144
Gln Arg Met Leu Cys Thr Arg Asp Ser Glu Cys Cys Gly Asp Gln Leu
        35                  40                  45

tgt gtc tgg ggt cac tgc acc aaa atg gcc acc agg ggc agc aat ggg      192
Cys Val Trp Gly His Cys Thr Lys Met Ala Thr Arg Gly Ser Asn Gly
    50                  55                  60

acc atc tgt gac aac cag agg gac tgc cag ccg ggg ctg tgc tgt gcc      240
Thr Ile Cys Asp Asn Gln Arg Asp Cys Gln Pro Gly Leu Cys Cys Ala
65                  70                  75                  80

ttc cag aga ggc ctg ctg ttc cct gtg tgc ata ccc ctg ccc gtg gag      288
Phe Gln Arg Gly Leu Leu Phe Pro Val Cys Ile Pro Leu Pro Val Glu
                85                  90                  95

ggc gag ctt tgc cat gac ccc gcc agc cgg ctt ctg gac ctc atc acc      336
Gly Glu Leu Cys His Asp Pro Ala Ser Arg Leu Leu Asp Leu Ile Thr
            100                 105                 110

tgg gag cta gag cct gat gga gcc ttg gac cga tgc cct tgt gcc agt      384
Trp Glu Leu Glu Pro Asp Gly Ala Leu Asp Arg Cys Pro Cys Ala Ser
        115                 120                 125

ggc ctc ctc tgc cag ccc cac agc cac agc ctg gtg tat gtg tgc aag      432
Gly Leu Leu Cys Gln Pro His Ser His Ser Leu Val Tyr Val Cys Lys
    130                 135                 140

ccg acc ttc gtg ggg agc cgt gac caa gat ggg gag atc ctg ctg ccc      480
Pro Thr Phe Val Gly Ser Arg Asp Gln Asp Gly Glu Ile Leu Leu Pro
145                 150                 155                 160

aga gag gtc ccc gat gag tat gaa gtt ggc agc ttc atg gag gag gtg      528
Arg Glu Val Pro Asp Glu Tyr Glu Val Gly Ser Phe Met Glu Glu Val
                165                 170                 175

cgc cag gag ctg gag gac ctg gag agg agc ctg act gaa gag atg gcg      576
Arg Gln Glu Leu Glu Asp Leu Glu Arg Ser Leu Thr Glu Glu Met Ala
            180                 185                 190

ctg ggg gag cct gcg gct gcc gcc gct gca ctg ctg gga ggg gaa gag      624
Leu Gly Glu Pro Ala Ala Ala Ala Ala Ala Leu Leu Gly Gly Glu Glu
        195                 200                 205

att                                                                  627
Ile

<210>  6
<211>  209
<212>  PRT
<213>  Homo sapiens

<400>  6

Arg Arg Ser His Glu Cys Ile Ile Asp Glu Asp Cys Gly Pro Ser Met
1               5                   10                  15

Tyr Cys Gln Phe Ala Ser Phe Gln Tyr Thr Cys Gln Pro Cys Arg Gly
            20                  25                  30

Gln Arg Met Leu Cys Thr Arg Asp Ser Glu Cys Cys Gly Asp Gln Leu
        35                  40                  45

Cys Val Trp Gly His Cys Thr Lys Met Ala Thr Arg Gly Ser Asn Gly
    50                  55                  60

Thr Ile Cys Asp Asn Gln Arg Asp Cys Gln Pro Gly Leu Cys Cys Ala
65                  70                  75                  80

Phe Gln Arg Gly Leu Leu Phe Pro Val Cys Ile Pro Leu Pro Val Glu
                85                  90                  95

Gly Glu Leu Cys His Asp Pro Ala Ser Arg Leu Leu Asp Leu Ile Thr
            100                 105                 110

Trp Glu Leu Glu Pro Asp Gly Ala Leu Asp Arg Cys Pro Cys Ala Ser
        115                 120                 125

Gly Leu Leu Cys Gln Pro His Ser His Ser Leu Val Tyr Val Cys Lys
    130                 135                 140

Pro Thr Phe Val Gly Ser Arg Asp Gln Asp Gly Glu Ile Leu Leu Pro
145                 150                 155                 160

Arg Glu Val Pro Asp Glu Tyr Glu Val Gly Ser Phe Met Glu Glu Val
                165                 170                 175

Arg Gln Glu Leu Glu Asp Leu Glu Arg Ser Leu Thr Glu Glu Met Ala
            180                 185                 190

Leu Gly Glu Pro Ala Ala Ala Ala Ala Ala Leu Leu Gly Gly Glu Glu
        195                 200                 205

Ile

<210>  7
<211>  8
<212>  PRT
<213>  Artificial

<220>
<223>  Synthetic

<400>  7

Ala Pro Ala Pro Thr Ala Thr Ser
1               5


<210>  8
<211>  662
<212>  DNA
<213>  Artificial

<220>
<223>  Synthetic

<400>  8
tgtatcgtct gcgcgttaag atccaagcca ccccggaatt ccggtagagc accatggggc      60

ggcttggggc caccctgctg tgcctgctgc tggcggcggc ggtccccacg gcccccgcgc     120

ccgctccgac ggcgacctcg tctgtgggag acgaagaagg cagaaggagc cacgagtgca     180

tcatcgacga ggactgtggg cccagcatgt actgccagtt tgccagcttc cagtacacct     240

gccagccatg ccggggccag aggatgctct gcacccggga cagtgagtgc tgtggagacc     300

agctgtgtgt ctggggtcac tgcaccaaaa tggccaccag gggcagcaat gggaccatct     360

gtgacaacca gagggactgc cagccggggc tgtgctgtgc cttccagaga ggcctgctgt     420

tccctgtgtg cacacccctg cccgtggagg gcgagctttg ccatgacccc gccagccggc     480

ttctggacct catcacctgg gagctagagc ctgatggagc cttggaccga tgcccttgtg     540

ccagtggcct cctctgccag ccccacagcc acagcctggt gtatgtgtgc aagccgacct     600

tctaagctct agactagatg actaacgttt aaacccgctg atcagcctcg actgtgcctt     660

ct                                                                    662


<210>  9
<211>  183
<212>  PRT
<213>  Artificial

<220>
<223>  Synthetic

<400>  9

Met Gly Arg Leu Gly Ala Thr Leu Leu Cys Leu Leu Leu Ala Ala Ala
1               5                   10                  15


Val Pro Thr Ala Pro Ala Pro Ala Pro Thr Ala Thr Ser Ser Val Gly

20                  25                  30

Asp Glu Glu Gly Arg Arg Ser His Glu Cys Ile Ile Asp Glu Asp Cys
        35                  40                  45

Gly Pro Ser Met Tyr Cys Gln Phe Ala Ser Phe Gln Tyr Thr Cys Gln
    50                  55                  60

Pro Cys Arg Gly Gln Arg Met Leu Cys Thr Arg Asp Ser Glu Cys Cys
65                  70                  75                  80

Gly Asp Gln Leu Cys Val Trp Gly His Cys Thr Lys Met Ala Thr Arg
                85                  90                  95

Gly Ser Asn Gly Thr Ile Cys Asp Asn Gln Arg Asp Cys Gln Pro Gly
            100                 105                 110

Leu Cys Cys Ala Phe Gln Arg Gly Leu Leu Phe Pro Val Cys Thr Pro
        115                 120                 125

Leu Pro Val Glu Gly Glu Leu Cys His Asp Pro Ala Ser Arg Leu Leu
    130                 135                 140

Asp Leu Ile Thr Trp Glu Leu Glu Pro Asp Gly Ala Leu Asp Arg Cys
145                 150                 155                 160

Pro Cys Ala Ser Gly Leu Leu Cys Gln Pro His Ser His Ser Leu Val
                165                 170                 175

Tyr Val Cys Lys Pro Thr Phe
            180

<210>  10
<211>  432
<212>  DNA
<213>  Artificial

<220>
<223>  Synthetic

<400>  10
gaattccggt agagcaccat ggaagctgcc gttgccgcac ctagaccacg ccttctgttg      60

ctggtactcg cagcggctgc tgccgcagca gcggctttgc tccctggagc cactgccctg     120

caatgcttct gtcacctgtg taccaaggac aatttcacgt gtgtgacaga tggcctgtgc     180

tttgtctcag tgaccgagac aacagacaag gtgatccaca actccatgtg cattgccgag     240

attgacctga taccccggga taggccgttt gtgtgtgccc catccagcaa aactgggagt     300

gtcaccacta cctactgttg caatcaggat cactgcaaca agatcgagct ccctacaacc     360

gtgaaaagct ctccaggtct tggccccgtt gaactgggac ccgggcatca ccaccatcat     420

cattaatcta ga                                                         432

<210>  11
<211>  134
<212>  PRT
<213>  Artificial

<220>
<223>  Synthetic

<400>  11

Met Glu Ala Ala Val Ala Ala Pro Arg Pro Arg Leu Leu Leu Leu Val
1               5                   10                  15

Leu Ala Ala Ala Ala Ala Ala Ala Ala Ala Leu Leu Pro Gly Ala Thr
            20                  25                  30

Ala Leu Gln Cys Phe Cys His Leu Cys Thr Lys Asp Asn Phe Thr Cys
        35                  40                  45

Val Thr Asp Gly Leu Cys Phe Val Ser Val Thr Glu Thr Thr Asp Lys
    50                  55                  60

Val Ile His Asn Ser Met Cys Ile Ala Glu Ile Asp Leu Ile Pro Arg
65                  70                  75                  80

Asp Arg Pro Phe Val Cys Ala Pro Ser Ser Lys Thr Gly Ser Val Thr
                85                  90                  95

Thr Thr Tyr Cys Cys Asn Gln Asp His Cys Asn Lys Ile Glu Leu Pro
            100                 105                 110

Thr Thr Val Lys Ser Ser Pro Gly Leu Gly Pro Val Glu Leu Gly Pro
        115                 120                 125

Gly His His His His His
    130